# EUROPÄISCHE PATENTANMELDUNG

(11) **EP 1 002 513 A1**
(43) Veröffentlichungstag der Anmeldung: **24.05.2000**
(21) Anmeldenummer: 99122195.3
(22) Anmeldetag: 06.11.1999
(51) Int. Cl.: A61K 6/033

(54) **Polyalkensäuren enthaltende Calcium-Phosphatzemente**

(30) Priorität: 21.11.1998 DE 19853832
(71) Anmelder: MERCK PATENT GmbH, 64293 Darmstadt (DE)
(72) Erfinder: Wenz, Robert, Dr., 61206 Wöllstadt (DE); Boltong, Trudy, 6109 AH Oheenlaak (NL)

(57) **Zusammenfassung**

Vorliegende Erfindung betrifft Calcium-Phosphat-Zemente mit verbesserten Eigenschaften, die mit wässrigen Polyalkensäuren angemischt werden für die Anwendung in Zahnzementen, Knochenzementen, Knochenersatzmaterialien, Knochenfüllstoffen, Knochenklebstoffen und als Trägermaterialien für Wirkstoffe oder Wachstumsfaktoren oder für andere medizinische und/oder technische Anwendungen.

## Beschreibung

Die vorliegende Erfindung betrifft biologisch abbaubare Calciumphosphat-Zemente, welche aus Calcium, Phosphor und Polyalkensäuren bestehen, und die für die Anwendung in Zahnzementen, Knochenzementen, Knochenersatzmaterialien, Knochenfüllstoffen, Knochenklebstoffen und als Trägermaterialien für Wirkstoffe oder Wachstumsfaktoren oder für andere medizinische und/oder technische Anwendungen dienen können.

Die Hauptkomponenten kalzifizierter Gewebe sind Calciumphosphate, im besonderen vom apatitartigen Typ (Driessens, Verbeek, Biominerals, CRC Press, Boca Raton, FL, USA, 1990). Aus diesem Grund bestehen die meisten Knochenersatzmaterialien aus Calciumphosphaten (Hollinger et al Clin. Orthop. Relat. Res. 324, 55-65, 1996). Einige Calciumphosphate werden in Form von Keramiken angewendet z. B. Hydroxylapatit (HA) oder β-Tricalciumphosphat (β-TCP). Ihr Vorteil liegt in der sehr guten Bioverträglichkeit sowohl im Knochengewebe als auch im Weichteilmantel. Dicht gesintertes HA scheint nicht biologisch abbaubar zu sein, wohingegen β-TCP als bioresorbierbar und osteokonduktiv gilt; die Kombination dieser Eigenschaften wird als Osteotransduktivität bezeichnet.

Calcium-Phosphat-Keramiken werden als biokompatible Knochenersatzmaterialien eingesetzt. Sie werden entweder in Form von Granulaten oder präformierten Formkörpern hergestellt. Die Granulatform bereitet wegen ihrer Mobilität am Implantationsort Probleme und die Formkörper haben aufgrund ihrer Sprödigkeit und wegen der oftmals nicht gegebenen Formschlüssigkeit am Implantationsort Nachteile. Um diese Nachteile zu lösen wurden mehrere Lösungsansätze unternommen (beispielsweise beschrieben von Driessens, Boltong, Wenz, in der P 98 13 614). Die dort beschriebene Erfindung betrifft biologisch abbaubare Calciumphosphat-Zemente, insbesondere Mischungen aus calciumphosphathaltigen Pulvern unterschiedlicher stöchiometrischer Zusammensetzung mit verbesserten Eigenschaften. Die erfindungsgemäßen Mischungen enthalten alle Tricalciumphosphat (TCP) und eine oder mehrere andere phosphathaltige anorganische Verbindungen in unterschiedlicher Zusammensetzung, wobei der TCP-Anteil in einem wohl definierten Partikelgrößenbereich vorliegt. Ihr Vorteil liegt in der guten Applizierbarkeit und des formschlüssigen Ausfüllens irregulärer Kavitäten. Nachteile liegen in ihrer Sprödigkeit und weniger guten Adhäsion am Knochen.

Die Hauptkomponenten solcher Calciumphosphatzementen (CPC) sind beispielsweise Tricalcium-phosphat (TCP), Dicalciumphosphat (DCP) und Tetracalciumphosphat (TTCP), welche in Anwesenheit von Wasser zu Hydroxylapatit reagieren, dem Endprodukt der Zementbildungs-Reaktion. Da derartig gebildetes Hydroxylapatit in wäßriger Umgebung entstanden ist, ähnelt es den biologischen Apatiten weit mehr als dem Hydroxylapatit, welches bei hohen Temperaturen erzeugt wird. Daher sind derartige Zemente osteotransduktiv und somit sehr gut zur Reparatur und Rekonstruktion von Knochen geeignet. Sie werden schnell in Knochenstrukturen integriert und danach durch die zelluläre Aktivität der Osteoblasten und Osteoklasten zu neuem Knochengewebe umgewandelt.

Solche Materialien (CPC) können leicht zu den gewünschten Formkörpern geformt werden, da sie zur Selbstaushärtung fähig sind. Dieses Selbstaushärten geschieht durch eine Abbindereaktion, welche in einem wässrigen Medium , z.B. Wasser, verdünnte wässrige Phosphorsäure oder sogar auch wässrige Lösungen, die Blut enthalten, die eingesetzten Calciumphosphate zu Hydroxylapatit (HA) umwandelt.

Neuerdings wurden auch Zemente entwickelt durch Anmischen von wässrigen Polymeren wie Polyalkensäuren, Polyvinylalkoholen oder auch Gelatine mit Mischungen aus Tetracalciumphosphat (Ca₄(PO₄)₂O) und Dicalciumphosphat CaHPO₄ (Miyazaki et al, Dent. Mater. 9, 41-45, 1993). Dadurch resultieren Zemente, die sehr hohe mechanische Stärken aufweisen. Nachteile der Kombination von Polyalkensäuren mit diesen Calciumphosphaten liegen jedoch in der überaus kurzen Verarbeitungszeit, die für medizinische und technische Anwendungen ungeeignet sind und die es unmöglich machten, Adhäsionstests zur Ermittlung der Klebewirkung am Knochen durchzuführen. Diese überaus schnelle Abbindezeiten sind auf die Reaktion zwischen den Polysäuren und den stark basischen Tetracalciumphosphaten zurückzuführen.

Es besteht also weiterhin der Bedarf an verbesserten als Biomaterialien einsetzbaren Zementen, die eine vernünftige Verarbeitungszeit ermöglichen und doch die gewünschten Eigenschaften wie geringe Sprödigkeit, gute Adhäsion am Knochen und auch hohe Bruchfestigkeit aufweisen.

Überraschenderweise wurde nun gefunden, dass verbesserte Bio-Zemente auf der Basis von α-Tricalciumphosphat, β-Tricalciumphosphat und/oder Calciumsulfat hergestellt werden können, wenn bei der Anmischung der Zementpaste eine wässrige Lösung von Polyalkensäuren bzw.deren Salze oder auch von Copolymeren von Polyalkensäuren mit weiteren Komponenten eingesetzt wird.

Gegenstand der Erfindung ist daher eine Zementpaste, bestehend aus einer flüssigen und pulvrigen Komponente, die zur Anwendung gemischt werden, dadurch gekennzeichnet, dass die flüssige Komponente aus niedrigmolekularen, wässrigen Polyalkensäuren oder deren Natrium- und/oder Kaliumsalze und/oder aus wässrigen Lösungen von Copolymeren der Alkensäuren mit Malonsäure oder Itaconsäure oder Allenen oder auch deren Mischungen, und das Zementpulver aus α-Tricalciumphosphat, β-Tricalciumphosphat und/oder Calciumsulfat (Dihydrat, Hemihydrat und/oder Anhydrid) besteht.

Das Zementpulver besteht also aus α- und/oder β-Tricalciumphosphat und/ oder Calciumsulfat und/oder deren Mischungen, wobei das Calciumsulfat als Dihydrat, Hemihydrat und/oder als Anhydrid vorliegen kann.

Die flüssige Komponente besteht vorzugsweise aus wässrigen, niedrigmolekularen Polyalkensäuren oder wässrigen Lösungen der entsprechenden Natrium- und/oder Kaliumsalze. Bevorzugt sind dabei wässrige Lösungen von Polyacrylsäure, Polymethacrylsäure oder auch Copolymere der Acrylsäure mit Malonsäure oder Itaconsäure. Weiterhin können auch deren Na- und/oder K-Salze als wässrige Lösungen eingesetzt werden. Niedrig-molekulare Polyalkensäuren oder Copolymere im Sinne dieser Erfindung bedeutet dabei **vorzugsweise < 8000 D. Besonders bevorzugt werden Produkte im Bereich von 1000 D bis 5000 D verwendet.**

Das Verhältnis Flüssigkeit zu Pulver variiert vorzugsweise zwischen 0,2 bis 10 Gramm/Gramm.

Mischungen aus α-TCP oder Calciumsulfat(―dihydrat,-hemihydrat, -anhydrat) härten, nachdem sie erfindungsgemäß mit den wässrigen Lösungen der Polyalkensäuren angemischt werden, spontan bei Raum- oder Körpertemperatur aus, aber mit vernünftigen Verarbeitungszeiten von länger als einer Minute. Der Mechanismus der Aushärtung erfolgt vermutlich durch eine Reaktion zwischen den Carboxylgruppen der Polyalkensäure und der Oberfläche des ausgehärteten Calcium-phospat/sulfat.

Um die Bioabbaubarkeit derartiger Zemente zu ermöglichen können andere anorganische Inhaltsstoffe wie HA, CaHPO₄, CaHPO₄·2H₂O, Ca₄(PO₄)₂O, CaNaPO₄, CaKPO₄, Ca₂NaK(PO₄)₂, CagMg(PO₄)₆ und andere Na, K, Mg und/oder Zn enthaltende Phosphate zugesetzt werden. Daraus hergestellte Zemente sind nur schwach sauer. In wässigen Medien steigt der pH-Wert der Oberfläche auf 6 innerhalb weniger Minuten, sodass diese Zemente während des Abbindevorganges nicht cytotoxisch wirken. Die Bioresorbierbarkeit stellt kein Problem dar, weil die anorganischen Bestandteile normalerweise als Ionen in den Körperflüssigkeiten enthalten sind und Polyalkensäuren verwendet werden deren Molekulargewicht (< 8000 D) eine renale Ausscheidung ermöglichen.

Die erfindungsgemäßen Biozemente weisen eine hohe Bruchfestigkeit auf. Die Scherkraft nach Verkleben von trabekulärem Knochen ist so groß oder größer als die von trabekulärem Knochen selbst. Obwohl die Bruchfestigkeit von Zementen beschrieben in der P 98 13 614, welche die derzeit festesten Calciumphosphatzemente darstellen, schon sehr hoch ist (≅ 200 Nmm), so ist die Schlagzähigkeit der Polyalkenoat-Calciumphosphatzemente 50 bis 100% höher als diese.

Auch nach Immersion der ausgehärteten Zemente über längere Zeit in Ringer-Lösung bleibt das α-TCP erhalten. Dies ist kein Nachteil, denn α-TCP und Calciumsulfat werden biologisch abgebaut. Die Abbaugeschwindigkeit als auch die Aushärtezeit derartiger Zemente kann durch Variation des α-TCP/β-TCP Verhältnisses im Zementpulver und durch Hinzufügen variabler Anteile von Füllstoffen wie CaHPO₄, CaHPO₄·2H₂O, HA, CaCO₃, CaNaPO₄, CaKPO₄, Ca₂NaK(PO₄)₂, carbonisiertem Apatit, Spochiosit, Chlorapatit, Ca₁₀Na(PO₄)₇, Ca₅Mg₄(PO₄)₆, CaZn₂(PO₄)₃ sowie von Mg- oder Zn-phosphaten, beeinflusst werden.

Die Zemente nach vorliegender Erfindung können als bioresorbierbare und sogar osteotransduktive Zemente verwendet werden für die Anwendung als Knochenersatzmaterial zur Auffüllung knöcherner Defekte, zur Alveolarkammaugmentation, Kiefer- und Gaumenplastiken, Trümmerfrakturen, Anwendungen in der Mund-, Kiefer- und Gesichtschirurgie, Vertebroplastie und Wirbelkörperfusion, endontotische Füllmaterialien, Wurzelzement, Regeneration von Knochenverlusten periodontitischer Genese oder auch zur Freisetzung von Wirkstoffen.

Die Implantation von Biomaterialien in den menschlichen oder tierischen Körper beinhaltet immer das Risiko der Besiedlung dieser unbelebten Materialien mit Keimen, weil diese Materialien zunächst keine Gefäßversorgung haben und damit vom Immunsystem nicht geschützt werden können. Deshalb ist es wünschenswert, den Biomaterialien zu ihrem eigenen temporären Schutz vor Keimbesiedlung Antibiotika z. B. aus der Aminoglycosidreihe wie Gentamicin, oder Cefazolin, Clindamycin-palmitat, insbesondere Clindamycin-phosphat oder Desinfektionsmittel zuzusetzen um eine Keimbesiedlung während der Implantation zu vermeiden.

Ferner ist es wünschenswert in resorbierbare Biozemente weitere pharmazeutische Wirkstoffe beizumischen, welche die verschiedensten Wirkungen besitzen, z.B. die zelluläre Aktivität des den Zement umgebenden Knochens erhöhen, im Sinne einer gesteigerten Resorption des Zementes und Ersatz dessen durch körpereigenen Knochen bzw. Bildung eines Komposites aus körpereigenem Knochen und nicht resorbierten Anteilen des Zementes oder Wirkstoffe im Sinne von Chemotherapeutika, welche die Auslockerung einer stabilisierenden Zementplombe nach Tumorresektion durch in der Nähe verbliebene Tumorzellen verhindern.

Beispiele solcher geeigneten pharmazeutischen Wirkstoffe sind Wachstumsfaktoren, wie FGF (Fibroblast Growth Factor), BMP (Bone Morphogenetic Protein) oder TGF-β (Tissue Growth Factor) oder andere Wirkstoffe wie Prostaglandine oder Stoffe, die den Prostaglandinstoffwechsel beeinflussen, Wirkstoffe, die mit dem Stoffwechsel der Schilddrüsen oder Nebenschildrüsen interagieren oder auch Chemotherapeutika, wie z.B. Metothrexat.

Gegenstand der Erfindung sind somit auch Mischungen, die zusätzlich einen oder mehrere pharmazeutische Wirkstoffe oder ein oder mehrere Desinfektionsmittel enthalten.

Vorzugsweise enthalten die erfindungsgemäßen Zemente bis zu 30 Gewichtsanteilen an einem oder mehreren Antibiotika oder bis zu 10 Gewichtsprozenten eines oder mehrerer Wachstumsfaktoren.

Zur Anwendung werden dann also die pulvrigen Komponenten mit der flüssigen Komponente angemischt, damit es dann zur Abbindung bzw. Bildung von Apatitstrukturen oder apatitähnlicher Massen kommt. Dadurch werden nach der Anmischung der Pulvergemische mit den wäßrigen Polymerflüssigkeiten vorteilhafte Eigenschaften erhalten. Diese Eigenschaften sind dadurch charakterisiert, dass die nach der Anmischung von fester und flüssiger Phase erhaltene Paste in temperaturabhängiger Weise bestimmte Verarbeitungsmöglichkeiten wie Modellierung und Injizierbarkeit in bestimmten Zeitintervallen ermöglicht. Prinzipiell können die Zusatzstoffe, wie pharmakologische Wirkstoffe oder Zusatzstoffe nicht nur dem TCP-Pulver beigemischt werden, sondern können auch in wässriger Lösung dem anzurührenden Biozement zugesetzt werden. Dieser liegt danach als sämige Suspension oder Paste vor und kann leicht in die vorgesehenen Orte oder defekten Knochenstrukturen eingebracht werden.

Somit ist auch Gegenstand der Erfindung eine entsprechende Mischung in Form einer wässrigen Lösung, Paste oder Suspension sowie ihre Verwendung zur Herstellung von biologisch abbaubaren implantierbaren synthetischen Knochenmaterialien.

Gegenstand der Erfindung sind ebenso biologisch abbaubare Zemente, dadurch gekennzeichnet, dass sie aus Calcium, Phosphor und Polyalkensäuren bestehen, hergestellt aus einer ausgehärteten Mischung gemäß einem der Ansprüche 1 bis 6.

Ferner ist Gegenstand der Erfindung die Verwendung von erfindungsgemäßen Zementpasten als bioresorbierbare und osteotransduktive Zemente für die Anwendung in Zahnzementen, Knochenzementen, Knochenersatzmaterialien, Knochenfüllstoffen, Knochenklebstoffen oder auch als Trägermaterialien für Wirkstoffe oder Wachstumsfaktoren.

In einer bevorzugten Ausführungsform dieser Erfindung liegt die Zementmischung in Form eines gebrauchsfertigen Sets aus zwei oder mehr getrennten Komponenten vor, dessen eine Komponente die flüssige Komponente und eine andere Komponente die Pulverkomponente beinhaltet. Bei Bedarf werden die Komponenten dann vermischt.

Die erfindungsgemäßen angerührten und abbindenden Mischungen zeichnen sich insbesondere durch eine angestrebte hohe Schlagzähigkeit und guter Bruchfestigkeit aus. Die Aushärtungszeiten sind wesentlich kürzer als bei den bisher verwendeten Zementen, jedoch lang genug, um vernünftige Verarbeitungszeiten zu haben. Auch ohne weitere Ausführungen wird davon ausgegangen, dass ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Die vollständige Offenbarung aller vor- und nachstehend aufgeführten Anmeldungen und Veröffentlichungen sind durch Bezugnahme in diese Anmeldung eingeführt.

Die folgenden Beispiele sollen die Erfindung näher erläutern.

### Beispiel 1

Eine 25%ige wässrige Polyacrylsäurelösung mit einem MW von ca. 3500 D wurde als Flüssigkeit zur Zementanmischung benutzt. Das benutzte α-TCP wurde zu einem Pulver einer mittleren Partikelgröße vom 10 µm vermahlen. Das verwendete Flüssigkeit/Pulver Verhältnis betrug 0,5g/0,5g. Die pulvrige Komponente wird dann mit der Flüssigkeit angerührt und gemischt. Die beginnende (initiale) Aushärtung (tᵢ) und die Zeit bis zur Erreichung der Endhärte (t_{f}) wurden bei Raumtemperatur (20±1°C) nach ASTM-Norm mittels Gilmoore-Nadeln bestimmt. tᵢ betrug 2 Min. 25 Sek. und t_{f} 6 Min. 30 Sek. Die Kompressionsfestigkeit nach Immersion in Ringer-Lösung betrug nach 1 Tag 18±2 und nach 4 Tagen 43±4 MPa.

Die Bestimmung der Kompressionsfestigkeit erfolgte mit einer Materialprüfmaschine Lloyd Typ LR50K nach gewünscht langer Immersion in Ringer-Lösung.

### Beispiel 2:

Verwendung der gleichen Polyacrylsäurelösung wie in Beispiel 1. Verwendung von β-TCP der mittleren Partikelgröße 10 µm. Flüssigkeits/Pulververhältnis 0,3g/g. Bei 20°C beträgt die initiale Aushärtezeit 4 Min 30 Sek., die finale Aushärtezeit 26,5 Minuten. Die Druckfestigkeit nach Immersion in Ringer-Lösung nach 1 Tag beträgt 1,5±0,1 MPa.

### Beispiel 3:

Man mischt eine Polyacrylsäurelösung aus Beispiel 1 mit α-TCP (mittlere Partikelgröße von 10µm). Das Flüssigkeits/Pulververhältniss wird folgendermaßen variiert: 0,5, 0,6, 0,65, 0,7 und 0,8 g/g. Die initialen und finalen Aushärtezeiten sowie die Druckfestigkeiten nach einem Tag Immersion in Ringer-Lösung sind in untenstehender Tabelle 1 zusammengefasst.

**Tab.1**

| Aushärtezeiten tᵢ und t_{f} in Minuten, Druckfestigkeit (Cs) nach 1 Tag in MPa, als Funktion des Flüssigkeits/Pulververhältnisses (F/P) | | | |
|---|---|---|---|
| F/P | tᵢ | t_{f} | Cs |
| 0,5 | 2,25 | 6,5 | 18 |
| 0,6 | 2,25 | 6,5 | 21 |
| 0,65 | 2,75 | 7 | --- |
| 0,7 | 2,75 | 8 | 14 |
| 0,8 | 4,5 | 14 | 11 |

### Beispiel 4:

Als Pulverkomponente wurden 84% α-TCP und 16% CaCO₃ verwendet und mit einer Polyacrylsäurelösung beschrieben in Beispiel 1 gemischt. Es wurde eine Paste im F/P-Verhältnis von 0,6g/g hergestellt. Es resultierten Werte von tᵢ = 2,75 Min. und t_{f} = 8,5 Min.

### Beispiel 5:

Als Pulverkomponente wurde hier eine Mischung von 62,5% α-TCP, 26,8% DCP, 8,9% CaCO₃ und 1,8% HA verwendet. Dieses Pulvergemisch wurde mit einer wie in Beispiel 1 beschriebenen Polyacrylsäurelösung zu einer Paste angerührt mit einem F/P-Verhältnis von 0,6g/g. Die Werte für tᵢ betrug 5 Min. und für t_{f} 16 Min.

### Beispiel 6:

Zur Anmischung einer erfindungsgemäßen Paste wurde die Polyacrylsäurelösung aus Beispiel 5 mit der Pulverkomponente α-TCP gemischt. Das F/P-Verhältnis ist 0,5 und 0,6g/g (Beispiel 6a bzw. 6b).

Nach 6tägiger Immersion in Ringer-Lösung bei 37°C wurden die Druckfestigkeit Cs in MPa, die Schlagzähigkeit TG in Nmm bestimmt. Diese Werte wurden mit den korrespondierenden Werten für Biocement D aus der P 98 13 614 verglichen (siehe Tabelle 2).

**Tab. 2**

| Zement | F/P | Cs | TG |
|---|---|---|---|
| Biocement D---- | 60 | 230 | |
| 6a | 0,5 | 37 | 320 |
| 6b | 0,6 | 34 | 310 |

Es ist aus der Tabelle ersichtlich, dass die Zemente, die mit Polyacrylsäure und α-TCP hergestellt wurden, eine höhere Schlagzähigkeit als Biocement D aufweisen.

### Beispiel 7:

Zur Herstellung einer Paste wurde die gleiche Polyacrylsäurelösung wie in Beispiel 6 und als Pulverkomponente α-TCP verwendet mit einem F/P-Verhältnis von 0,5g/g.

Mit dieser Paste wurden jeweils 2 Würfel aus frischem trabekulärem Rinderknochen mit einer Kantenlänge von 2 cm miteinander verklebt. Nach Immersion der verklebten Würfel in 0,9%iger Kochsalzlösung bei 37°C wurde die Scherkraft bis zum Auseinanderbrechen der Klebefuge auf einer Instron-Materialprüfmaschine bei einer Vorschubgeschwindigkeit von 1mm/Minute gemessen. Die Adhäsionskraft der verklebten Würfel miteinander betrug 1,07 MPa. Bei der Benutzung von Biocement D als Kleber wurden 0,41 MPa bestimmt.

### Beispiel 8:

Man stellte eine Paste her unter Verwendung einer Polyacrylsäurelösung wie in Beispiel 7 und Calciumsulfat-Hemihydrat als Pulverkomponente. Das F/P-Verhältnis wurde auf 0,3g/g eingestellt. Als Aushärtezeiten wurden ti mit 2,25 und tf mit 3,5 Minuten bei Raumtemperatur (21°C) bestimmt.

Bei Herstellung einer Paste aus Calciumsulfat-Hemihydrat und Wasser bei einem F/P-Verhältnis von 0,3g/g war ti = 13,5 und tf = 16,5 Minuten (Raumtemperatur 21°C).

### Beispiel 9:

Eine erfindungsgemäße Paste wurde hergestellt aus der gleichen Polyacrylsäurelösung wie in Beispiel 8 und α-TCP als Pulverkomponente. Das F/P-Verhältnis war 0,6g/g.
Mit der hergestellten Paste wurden jeweils 2 Würfel aus frischem trabekulärem Rinderknochen mit einer Kantenlänge von 2 cm miteinander wie in Beispiel 7 verklebt. Die verklebten Würfel wurden 2, 5 und 24 Stunden bei 37°C in 0,9% Kochsalzlösung inkubiert. Die Scherkraft zum Zerreißen der Würfel wurde bestimmt und gegen Verklebung mit Biocement D verglichen (siehe Tabelle 3).

**Tabelle 3**

| Zunahme der Scherkraft in Abhängigkeit von der Zeit. Die Standardabweichung in Klammern. | | |
|---|---|---|
| Inkubationszeit | Polyacrylsäure/α-TCP | Biocement D |
| 2 Std. | 0,443 (0,247) | 0,179 (0,061) |
| 5 Std. | 0,337 (0,143) | 0,421 (0,319) |
| 24 Std. | 0,510 (0,224) | 0,728 (0,263) |

Aus Tabelle 3 ist zu entnehmen, dass die Paste aus Polyacrylsäure und α-TCP Klebungen schon nach 2 Stunden ermöglicht.

## Patentansprüche

1. Zementpaste, bestehend aus einer flüssigen und pulvrigen Komponente, die zur Anwendung gemischt werden, dadurch gekennzeichnet, dass die flüssige Komponente aus niedrig-molekularen, wässrigen Polyalkensäuren oder deren Natrium- und/oder Kaliumsalze und/oder aus wässrigen Lösungen von Copolymeren der Alkensäuren mit Malonsäure oder Itaconsäure oder Allenen oder auch deren Mischungen, und das Zementpulver aus α-Tricalciumphosphat, β-Tricalciumphosphat und/oder Caiciumsulfat (Dihydrat, Hemihydrat und/oder Anhydrid) besteht.

2. Zementpaste nach Anspruch 1, dadurch gekennzeichnet, dass als flüssige Komponente eine wässrige Polyacrylsäure mit einem Molekulargewicht < 8000 D oder deren Natrium- und/oder Kaliumsalze oder Copolymere der Acrylsäure und Malonsäure oder Acrylsäure und Itaconsäure verwendet werden.

3. Zementpaste nach Anspruch 1 oder 2, dadurch gekennzeichnet, dass das Flüssigkeit/Pulververhältnis 0,2 bis 10 g/g Pulver beträgt.

4. Zementpaste nach einem der Ansprüche 1 bis 3, dadurch gekennzeichnet, dass das Zementpulver mindestens einen Zusatz ausgewählt aus der Gruppe:
CaHPO₄, CaHPO₄·2H₂O, Ca₈(HPO₄)₂(PO₄)₄·5H₂O, CaCO₃, Ca₉(HPO₄)(PO₄)OH, carbonisiertem Apatit, Ca₁₀(PO₄)₆(OH₂), Ca₄(PO₄)₂O, CaNaPO₄, Ca₁₀Na(PO₄)₂, CaKPO₄, Ca₂NaK(PO₄)₂, Ca₅Mg₄(PO₄)₆, Ca₈Mg(PO₄)₆, Ca₂Zn₂(PO₄)₂, Ca₂PO₄Cl, Chlorapatit, und/oder Phosphate von Magnesium und Zink, enthält.

5. Zementpaste nach einem der Ansprüche 1 bis 4 zusätzlich enthaltend bis zu 30 Gewichtsanteilen eines Antibiotikums oder Gemische verschiedener Antibiotika.

6. Zementpaste nach einem der Ansprüche 1 bis 4 zusätzlich enthaltend bis zu 10 Gewichtsprozent eines oder mehrerer Wachstumsfaktoren.

7. Biologisch abbaubare Zemente, dadurch gekennzeichnet, dass sie aus Calcium, Phosphor und Polyalkensäuren bestehen, hergestellt aus einer ausgehärteten Mischung gemäß einem der Ansprüche 1 bis 6.

8. Verwendung von Zementpasten nach Anspruch 7 als bioresorbierbare und osteotransduktive Zemente für die Anwendung in Zahnzementen, Knochenzementen, Knochenersatzmaterialien, Knochenfüllstoffen, Knochenklebstoffen oder auch als Trägermaterialien für Wirkstoffe oder Wachstumsfaktoren.
